# EUROPEAN PATENT SPECIFICATION

(11) **EP 4 406 520 B1**
(45) Date of publication and mention of the grant of the patent: **02.09.2026**
(21) Application number: 24154383.4
(22) Date of filing: 29.01.2024
(51) Int. Cl.: A61F 5/37, G16H 20/30

(54) **SYSTEM FOR THE APPLICATION OF RESTRAINTS ON PATIENTS BY HEALTHCARE STAFF**
SYSTEM ZUR ANWENDUNG VON FESSELN AN PATIENTEN DURCH GESUNDHEITSPERSONAL
SYSTÈME D'APPLICATION DE CONTRAINTES SUR LES PATIENTS PAR LE PERSONNEL DE SANTÉ

(30) Priority: 27.01.2023 ES 202330126 U
(43) Date of publication of application: 31.07.2024
(73) Proprietor: Medi Care System, S.L.U., 08302 Mataró (ES)
(72) Inventor: MONTERO ALFONSO, Manuel, 08302 MATARÓ (ES)
(74) Representative: Manresa Medina, José Manuel

(56) References cited:
- CN-A- 106 898 067
- GB-A- 1 506 926
- US-A- 5 224 496
- US-A1- 2005 083 207
- US-A1- 2013 019 882
- US-A1- 2014 340 217

## Description

System for the application of restraints on patients by healthcare staff, comprising patient identification means, characterized by restraint means comprising identification and tracking means, and reading and transmitting data means for being used by healthcare staff, and further means, with which the patient is associated, for capturing patient's vital signs data which are sent to and analysed by central processor creating a pattern of the patient's behaviour, and wherein the patient identification means are readable by the reading means, which display such information to the healthcare staff and then sends such information to a central data processor that stores it, the identification and tracking means of the restraint means are readable by the reading means, which display such information to the healthcare staff and send it to the central processor that associates the patient identification means with the identification and tracking means of the restraint means and with the healthcare staff's means of reading and transmitting data in order to track the length of time of application of restraint means on the patient.

### BACKGROUND OF THE INVENTION

Different systems for the application of restraints on patients are known are known in the state of the art.

Thus, state of the art is CN106898067A "Positioning device verification method and system and treatment bed" of the year 2017, in the name of SHENYANG NEUSOFT MEDICAL SYS which refers to a positioning device verification method and system and a treatment bed. A device information reader is arranged on the treatment bed of an accelerator, and device identification information is set for a positioning device to be verified. The positioning device verification method comprises the steps that the treatment bed receives target patient verification information sent by a control console, wherein the verification information is stored in a database which can be accessed by the control console and includes the device identification information of a target patient positioning device; a device information reader of the treatment bed reads the device identification information of the positioning device to be verified; the treatment bed matches the read device identification information with the device identification information of the target patient positioning device, and a matching result is prompted. By adopting the positioning device verification method and system and the treatment bed, whether the positioning device is matched with a target patient or not can be correctly verified.

An additional system forming part of the prior art is disclosed by US 2005/083207 A1 (SMITH TOBY E [US] ET AL).

### BRIEF DESCRIPTION OF THE INVENTION

The present invention is framed within the sector of sanitary restraint systems for patients.

By way of introduction, restraint is understood to be the clinical/therapeutic system of immobilization or partial or total positioning of a person, which may be a system with elements and/or devices for restraint to the bed, to the chair-buttock, etc.

The use of mechanical and pharmacological restraints has been the subject of debate for a long time, but especially in recent years the controversy has increased, with numerous movements advocating the reduction and even the total elimination of restraints, both in the psychiatric and geriatric area. Although this desire is shared by the majority of patients' relatives or residents and also by an increasing number of health professionals, the reality indicates that it is difficult to find alternatives to restraints or to implement work methodologies that manage to reduce their use.

The present invention aims on the one hand to reduce the use of restraints, applying them in a rational and planned way, thus contributing to protect the dignity of the patients, the safety of the patient and of the professionals in order to achieve the "0" trend in the use of restraints.

On the other hand, the aim is to facilitate the work of the healthcare staff, allowing the physician to define a specific "restraint plan" for each patient, which can be executed safely and simply by the healthcare staff, using the management and prediction tools made available by this invention.

On the other hand, the aim is to legally protect the institutions and their professionals by creating an external, secure and inviolable record of the use of restraints for each patient admitted.

An object of the present invention is system for the application of restraints on patients by healthcare staff, comprising patient identification means, characterized by restraint means comprising identification and tracking means, and reading and transmitting data means for being used by healthcare staff, and further means, with which the patient is associated, for capturing patient's vital signs data which are sent to and analysed by central processor creating a pattern of the patient's behaviour, and wherein the patient identification means are readable by the reading means, which display such information to the healthcare staff and then sends such information to a central data processor that stores it, the identification and tracking means of the restraint means are readable by the reading means, which display such information to the healthcare staff and send it to the central processor that associates the patient identification means with the identification and tracking means of the restraint means and with the healthcare staffs means of reading and transmitting data in order to track the length of time of application of restraint means on the patient.

### PRACTICAL EMBODIMENT OF THE PRESENT INVENTION

Thus, in a practical realization, the health staff, faced with a serious problem-that requires the application of a restraint measure to a patient, would proceed as follows.

First of all, I would place the means of restraint on the patient, for example, a belt, wrist and ankle braces.

The data reader and data transmitter will then read the customer's identifier, display it on a screen and transmit it to the central processor that manages the data.

Then, the healthcare staff, by means of the reader, will read the means of identification of all the means of restraint applied to the patient, visualize them and send the data to the central processor that manages them, thus associating the patient's identifier with the means of restraint applied to the patient and with the healthcare staff who read them.

In this way, the length of time the patient being in the restraint means can be tracked and an alert given when the predetermined containment time period is approaching.

Optionally, the restraint means may comprise antibacterial and fire retardant properties. This is done to prevent infection and for fire prevention. In addition, since the restraint means has means of identification, this means that it is possible to follow its traceability and to know whether the restraint means has been cleaned or not, how long it has been in use (both in terms of its durability and whether it still retains its antibacterial properties), which healthcare staff have used it, etc.

For this system, an RFID tag has been chosen as the means of identification and tracking.

The use of RFIDs nowadays makes it possible to track the patient's position, i.e., to know the patient's position at all times so that the healthcare staff can act, wherever they are, when any action needs to be taken on the means of restraint (e.g., loosening them).

The central processor manages the information and displays it on a control panel for the medical staff. This allows the medical staff to monitor the patient remotely, thus facilitating quick reactions of the medical staff to certain warnings.

To facilitate the issue of data management and storage, it has been configured so that the collected data is located and processed in a cloud that distributes it to the control panels.

Another option is for the patient to be associated with a means of capturing vital signs data, which may or may not be in the means of restraint, for example, attached to the patient's skin, which are sent to the central processor that analyses them and creates a pattern of the patient's behaviour. In this way, patient behaviour can be predicted, and the restraints can be removed prematurely if it is detected that the client, in view of the patterns, will not generate a behaviour that requires the restraints.

It would also be possible to predict, with the data supplied to it, behaviours through the use of artificial intelligence.

Optionally, it includes a system of traceability of the restraint means when they are not in use, being able to control its status, activated or stored, in the laundry or its location within the centre. That is, if the restraint means are sent to the laundry, the number of times it goes to the laundry (and other additional data) is recorded, as well as when it returns, and where it is stored.

This is done in order to know exactly the available units of restraint equipment in use or free for use.

On the other hand, it is used to know the deterioration of the product, for example, the number of washings, time of use, time since its manufacture, etc. All these data influence the shelf life of the product, so the condition of the product is measured according to these parameters to ensure that the restraint means are always in good condition.

Finally, traceability is used to identify those that are lost, which represent a great economic drain on the coffers of health centres.

In addition, this benefits safety, because as the restraint media are used, taken to the laundry, stored, etc., the system has a life cycle algorithm that discounts the useful life of the restraint media to the point at which it is considered appropriate to change it and informs about it.

Thus, in a specific embodiment, when faced with a patient with one or more means of restraint (wristbands, anklets, ...), the first step would be to point the means of reading and transmitting data, for example, a pad reader, to the means of identification of the patient so that the health staff can know the person to whom the means of restraint are being applied.

Thus, the patient's data will be displayed on the pad screen.

It will then point the pad at the means of restraint which are identified by RFID tags in this embodiment, and which will display on the pad screen the means of restraint and the position of the restraints, e.g. anklets, wristbands, abdominal restraints, ....

These data are sent by the pad to the central computer, which sends different data to the client, such as the time the treatment has been applied, the time remaining and other data of interest.

The health staff may then decide to terminate the restraint and would therefore remove the means of restraint from the patient.

He would then send the restraint means to the laundry, and readers would determine their departure. Then when they returned, they would check in again.

Additionally, the central computer, using a shelf life algorithm, will calculate the shelf life of each restraint medium after each use on a patient and each wash in a laundry.

At the end of the useful life of the restraint means, it would be disposed of.

This traceability helps, as mentioned above, to know at all times where the restraint means is located, in order to be able to make forecasts, calculate when to renew them, or those that are not available because they are in the laundry.

It is also important to note that the healthcare staff can know where the patient is with the restraint devices, since if different readers are distributed at different points, they can record the patient's movements, and thus, when the central computer alerts the healthcare staff that the treatment must be changed, the healthcare staff will be able to reach the patient more quickly.

The present invention describes a new system for the application of restraints on patients by healthcare staff. The scope of the present invention is defined by the following claims.

## Claims

1. System for the application of restraints on patients by healthcare staff, comprising patient identification means, **characterized by**:
- restraint means comprising identification and tracking means, and
- reading and transmitting data means for being used by healthcare staff,
- means, with which the patient is associated, for capturing patient's vital signs data which are sent to and analysed by a central processor creating a pattern of the patient's behaviour.
and wherein
- the patient identification means are readable by the reading and transmitting data means, which display such information to the healthcare staff and then sends such information to the central processor that stores it,
- the identification and tracking means of the restraint means are readable by the reading and transmitting data means, which display such information to the healthcare staff and send it to the central processor that associates the patient identification means with the identification and tracking means of the restraint means and with the healthcare staff's reading and transmitting data means in order to track the length of time of application of restraint means on the patient.

2. System according to claim 1, **characterized in that** the restraint means comprise antibacterial and fire-retardant properties.

3. System according to claim 2, **characterized in that** the identification and tracking means are an RFID tag.

4. System according to claim 1, **characterized in that** the collected data are located and processed in a cloud that distributes them to control panels.

5. System according to claim 1, **characterized in that** it comprises a central processor that generates an alarm in case of certain events.

6. System, according to claim 1, **characterized in that** it comprises a system of traceability of the restraint means when they are not in use.

7. System according to claim 6, **characterized in that** it includes an algorithm for the calculation of the useful life of the restraint means, according to their wear.

## Patentansprüche

1. System, bei dem Pflegefachkräfte Fixierungen an Patienten anbringen, die Mittel zur Identifizierung des Patienten enthalten, **dadurch gekennzeichnet dass**:
- die Fixierungen Identifizierungs- und Ortungsmittel umfassen, sowie
- Mittel zum Auslesen und Übertragen von Daten, die von Pflegefachkräften verwendet werden,
- Mittel, die am Patienten angebracht werden, um dessen Vitalparameter zu erfassen; diese Daten werden an einen zentralen Prozessor übertragen und dort analysiert, wodurch ein Muster des Patientenverhaltens erstellt wird;
und wobei
- die Patientenidentifikationsmittel von den Lese- und Datenübertragungsmitteln gelesen werden können, die den Pflegefachkräften diese Informationen anzeigen und sie anschließend an den zentralen Prozessor senden, der diese speichert,
- die Identifizierungs- und Ortungsmittel der Fixierungen von den Mitteln der Lese- und Übertragungsdaten der Pflegefachkräfte ausgelesen werden können, die diese Informationen den Pflegefachkräften anzeigen und an den zentralen Prozessor senden, der die Patientenidentifikationsmittel mit den Identifizierungs- und Ortungsmitteln der Fixierungen und mit den Lese- und Übertragungsmitteln der Pflegefachkräfte verknüpft, um die Dauer der Verwendung der Fixierungen am Patienten zu erfassen.

2. System nach Anspruch 1, **dadurch gekennzeichnet, dass** die Fixierungen antibakterielle und flammhemmende Eigenschaften aufweisen.

3. System nach Anspruch 2, **dadurch gekennzeichnet, dass** es sich bei den Identifizierungs- und Ortungsmitteln um ein RFID-Tag handelt.

4. System nach Anspruch 1, **dadurch gekennzeichnet, dass** die erfassten Daten in einer Cloud gespeichert und verarbeitet werden, die sie an Bedienfelder weiterleitet.

5. System nach Anspruch 1, **dadurch gekennzeichnet, dass** es einen zentralen Prozessor enthält, der bei bestimmten Ereignissen einen Alarm auslöst.

6. System nach Anspruch 1, **dadurch gekennzeichnet, dass** es ein System zur Rückverfolgbarkeit der Fixierungen enthält, wenn diese nicht in Gebrauch sind.

7. System nach Anspruch 6, **dadurch gekennzeichnet, dass** es einen Algorithmus zur Berechnung der Lebensdauer der Fixierungen entsprechend ihrem Verschleiß enthält.

## Revendications

1. Système pour l'application de contraintes sur des patients par le personnel soignant, comprenant un moyen d'identification de patient, **caractérisé par**:
- un moyen de contraintes comprenant un moyen d'identification et de suivi, et
- un moyen de lecture et de transmission de données destiné à être utilisé par le personnel soignant,
- un moyen, auquel est associé le patient, destiné à capturer des données des signes vitaux du patient qui sont envoyées à un processeur central et analysées par ce dernier créant un profil du comportement du patient,
et dans lequel
- le moyen d'identification de patient peut être lu par le moyen de lecture et de transmission de données, lequel affiche ces informations à l'attention du personnel soignant puis envoie ces informations au processeur central qui les stocke,
- le moyen d'identification et de suivi du moyen de contraintes peut être lu par le moyen de lecture et de transmission de données, lequel affiche ces informations à l'attention du personnel soignant et les envoie au processeur central qui associe le moyen d'identification de patient au moyen d'identification et de suivi du moyen de contraintes et au moyen de lecture et de transmission de données du personnel soignant afin de suivre la durée d'application du moyen de contraintes sur le patient.

2. Système selon la revendication 1, **caractérisé en ce que** le moyen de contraintes comprend des propriétés antibactériennes et ignifuges.

3. Système selon la revendication 2, **caractérisé en ce que** le moyen d'identification et de suivi est une étiquette RFID.

4. Système selon la revendication 1, **caractérisé en ce que** les données collectées sont situées et traitées dans un nuage qui les distribue à des panneaux de commande.

5. Système selon la revendication 1, **caractérisé en ce qu'**il comprend un processeur central qui génère une alarme en cas de certains événements.

6. Système selon la revendication 1, **caractérisé en ce qu'**il comprend un système de traçabilité du moyen de contraintes lorsqu'il n'est pas en cours d'utilisation.

7. Système selon la revendication 6, **caractérisé en ce qu'**il inclut un algorithme pour le calcul de la durée de vie du moyen de contraintes, en fonction de son usure.
